# EUROPEAN PATENT APPLICATION

(11) **EP 3 561 499 A1**
(43) Date of publication of application: **30.10.2019**
(21) Application number: 17881855.5
(22) Date of filing: 27.10.2017
(51) Int. Cl.: G01N 27/416, G01N 33/483, G01N 33/497

(54) **EXHALATION SENSOR**

(30) Priority: 14.12.2016 JP 2016242431
(71) Applicant: NGK Spark Plug Co., Ltd., Nagoya-shi, Aichi 467-8525 (JP)
(72) Inventor: SHICHIDA, Takafumi, Nagoya-shi Aichi 467-8525 (JP); NISHIO, Kenji, Nagoya-shi Aichi 467-8525 (JP); UEKI, Masatoshi, Nagoya-shi Aichi 467-8525 (JP); INOUE, Tsuyoshi, Nagoya-shi Aichi 467-8525 (JP)
(74) Representative: J A Kemp LLP
(86) International application number: PCT/JP2017/038833
(87) International publication number: WO 2018/110117

(57) **Abstract**

Provided is an exhalation sensor that utilizes heat effectively and can operate with low power consumption. The exhalation sensor 1 has a surface layer 4 on its surface that faces a sensor main body 37, and the performance of the surface layer 4 to reflect radiant heat is higher than that of a housing 3. Specifically the radiant heat reflectivity of the surface layer 4 is higher than that of the housing 3. Therefore, the surface layer 4 can reflect radiant heat emitted from the sensor main body 37 more efficiently than the housing 3. The radiant heat emitted from the sensor main body 37 is less likely to escape to the outside of the housing 3, so that the heat generated by a heater 29c of the sensor main body 37 can be efficiently stored within the housing 3. This can reduce the power consumed by the heater 29c to heat a conversion section 21 and a sensing section 29a to their operating temperatures.

## Description

### TECHNICAL FIELD

The present disclosure relates to an exhalation sensor that detects the concentration of a specific gas component contained in exhaled breath.

### BACKGROUND ART

One known sensor for diagnosis of, for example, asthma measures NOx contained at a very low concentration (at a level of several ppb to several hundreds of ppb) in exhaled breath (see Patent Document 1).

In this sensor, a conversion section including a PtY (Pt-bearing zeolite) catalyst for converting NO in exhaled breath to NO₂ and a sensing section including a mixed potential sensor element for detecting NO₂ are formed as a single unit using a ceramic stacking technique.

In this sensor, the optimal operating temperature of the catalyst differs from the optimal operating temperature of the sensor element. Therefore, a heater for heating the catalyst is disposed in the conversion section, and a heater for heating the sensor element is disposed in the sensing section. These heaters are controlled separately to different temperatures.

### PRIOR ART DOCUMENT

### PATENT DOCUMENT

Patent Document 1: U.S. Patent Application Publication No. 2015/0250408

### SUMMARY OF THE INVENTION

### PROBLEM TO BE SOLVED BY THE INVENTION

In the case where the exhalation sensor is made compact, for example, for portability, a battery for the exhalation sensor is also required to be compact. In such a case, the conventional technique raises problems such as limitation on the time over which the exhalation sensor is driven by the battery (hereinafter referred to as the driven time of the exhalation sensor).

Namely, in the case where the battery for supplying electric power to the heaters is made compact, in general, the capacity of the battery decreases, which raises a problem in that the conditions of heating by the heaters (e.g., heating time) are restricted. There is therefore a need to reduce the power consumption of the heaters, etc. as much as possible.

The present disclosure has been made in view of the foregoing circumstances, and it is an object to provide an exhalation sensor in which heat is utilized effectively so that the exhalation sensor can operate with low power consumption.

### MEANS FOR SOLVING THE PROBLEMS

(1) An exhalation sensor of a first aspect of the present disclosure comprises a sensor main body including a sensor unit having a chamber into which exhaled breath is to be introduced and a sensing section whose electrical characteristic changes with the concentration of a specific gas component in the chamber, and a heater for heating the sensing section, and further comprises a housing disposed so as to surround an outer circumference of the sensor main body.
   The housing includes a surface layer on a surface thereof that faces the sensor main body, and the surface layer is higher than the housing in terms of the performance of reflecting radiant heat emitted from the sensor main body.
   As described above, in the exhalation sensor of the first aspect, the surface layer on the side facing the sensor main body has a radiant heat reflecting performance higher than that of the housing. Specifically, the radiant heat reflectivity of the surface layer is higher than that of the housing. Therefore, the surface layer can reflect the radiant heat emitted from the sensor main body more efficiently than the housing.
   The radiant heat emitted from the sensor main body is less likely to escape to the outside of the housing, and therefore the heat generated by the heater can be efficiently stored within the housing.
   Notably, when the heat generated by the heater is less likely to escape to the outside of the housing, the temperature inside the housing does not easily decrease, so that the power consumed by the heater to heat the sensing section to its operating temperature can be reduced.
   As described above, in the first aspect, since heat can be effectively utilized, a remarkable effect of reducing the power consumption can be achieved.
   In particular, in the case where the exhalation sensor is compact and portable, the capacity of the battery used is also small. Therefore, by reducing the power consumption of the heater, the consumption of energy (i.e., electric power) stored in the battery can be reduced. Namely, since the battery runtime (so-called life) can be extended, the effect is remarkable.
   In the first aspect, the time from when the exhalation sensor is turned on to when the exhalation sensor starts operating can be shortened. Specifically, the time from when the heater is energized until a prescribed temperature (i.e., the operating temperature) is reached can be shortened. Therefore, the exhalation sensor has an advantage of improved startup performance.
   The radiant heat reflectivity is the ratio of the energy Er reflected by the reflecting surface to the total energy E of the radiant heat emitted (= Er/E).
(2) In a second aspect of the present disclosure, the exhalation sensor may further comprise an adjustment unit including a chamber into which the exhaled breath is to be introduced and a conversion section that converts a first gas component contained in the exhaled breath introduced into the chamber of the adjustment unit to a second gas component; and a heater for heating the conversion section. Further, the chamber of the sensor unit may be configured such that the exhaled breath passing through the chamber of the adjustment unit is introduced into the chamber of the sensor unit, and the sensing section of the sensor unit may be configured such that its electrical characteristic changes with the concentration of the second gas component in the exhaled breath introduced from the chamber of the adjustment unit.
   In the second aspect of the present invention, the conversion section of the adjustment unit can convert the first gas component contained in the exhaled breath introduced into the chamber (e.g., the first chamber) of the adjustment unit to the second gas component. The exhaled breath ion passing through the chamber of the adjustment unit can be introduced into the chamber (e.g., the second chamber) of the sensor unit. In the sensing section, its electrical characteristic changes with the concentration of the second gas component in the exhaled breath introduced.
   As described above, in the second aspect, the conversion section converts the first gas component contained in the exhaled breath to the second gas component, and the electrical characteristic of the sensing section changes with the concentration of the second gas component. Therefore, the concentration of the specific gas component can be detected based on the electrical characteristic changed with the concentration of the second gas component.
(3) In a third aspect of the present disclosure, a single heater may be used as the heater for heating the conversion section and the heater for heating the sensing section.
   In the case where a single heater is used as in the third aspect, the device structure can be simplified, and the battery can be made compact.
   In particular, in the case where a single heater is used, consumption of electric power of the battery can be suppressed as compared with the case where a plurality of heaters are used for heating. Therefore, even when the capacity of the battery is small, it is possible to mitigate the limitation on the driven time of the exhalation sensor, etc.
(4) In a fourth aspect of the present disclosure, the housing may be made of a resin.
   In the case where the housing is made of a resin, advantageously, the housing has a lighter weight and an enhanced heat insulating performance as compared with the case where the housing is made of, for example, a metal. In particular, in the case where the housing has an enhanced heat insulating performance, the temperature inside the housing does not easily decrease, so that the power consumption of the heater can be further reduced.
(5) In a fifth aspect of the present disclosure, the surface layer may be a metal layer.
   In the case where the surface layer is a metal layer, its radiant heat reflectivity is generally about 0.5 to about 0.9 and is higher than that of the resin (e.g., 0.1 to 0.3). This is advantageous because the radiant heat is less likely to escape to the outside. As a result, the power consumption of the heater can be further reduced.
(6) In a sixth aspect of the present disclosure, the surface layer may be a plating layer.
   The metallic plating layer can reflect the radiant heat efficiently. As a result, the power consumption of the heater can be further reduced.
(7) In a seventh aspect of the present disclosure, the surface layer may be a film layer formed of a metal film.
   The film layer formed of the metal film can reflect the radiant heat efficiently. As a result, the power consumption of the heater can be further reduced.
(8) In an eighth aspect of the present disclosure, the surface layer may have a radiant heat reflectivity of 0.5 or more.

When the radiant heat reflectivity of the surface layer is 0.5 or more, the radiant heat can be reflected efficiently. As a result, the power consumption of the heater can be further reduced.

### BRIEF DESCRIPTION OF THE DRAWINGS

FIG. 1 is a plan view showing an exhalation sensor of a first embodiment.
FIG. 2 is a cross-sectional view showing a cross section (cross section taken along line A-A in FIG. 1) of the exhalation sensor of the first embodiment.
FIG. 3 is an enlarged cross-sectional view showing a cross section (cross section taken along line A-A in FIG. 1) of a sensor main body of the first embodiment.
FIG. 4 is a cross-sectional view showing a cross section (cross section taken along line B-B in FIG. 1) of the exhalation sensor of the first embodiment.
FIG. 5 is a cross-sectional view partially showing a housing of the first embodiment and a surface layer thereof, the housing and the surface layer being cut in their thickness direction.
FIG. 6 is a cross-sectional view showing an exhalation sensor of a second embodiment, the exhalation sensor being cut along an inlet etc.
FIG. 7 is a cross-sectional view showing part of an exhalation sensor of a third embodiment, the exhalation sensor being cut along an inlet etc.

### MODES FOR CARRYING OUT THE INVENTION

Embodiments of an exhalation sensor to which the present disclosure is applied will next be described with reference to the drawings.

### [1. First embodiment]

### [1-1. Overall structure of exhalation sensor]

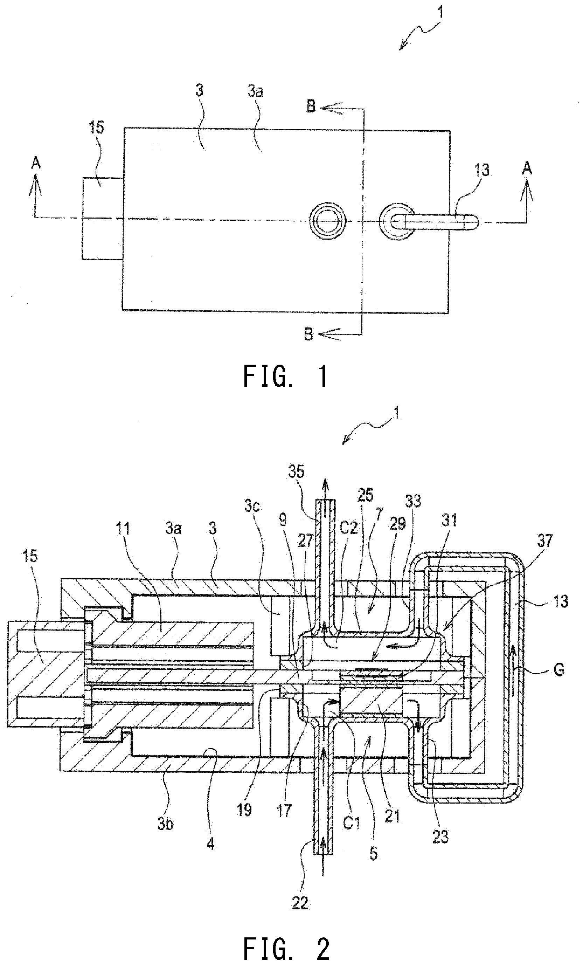

As shown in FIGS. 1 and 2, in an exhalation sensor 1 of a first embodiment, an adjustment unit 5, a sensor unit 7, a ceramic wiring board 9, and a first connector portion 11 are contained within a housing 3. The exhalation sensor 1 includes a gas flow pipe 13 that connects the adjustment unit 5 to the sensor unit 7, and a second connector portion 15 connected to the first connector portion 11.

This exhalation sensor 1 is driven by electric power supplied from, for example, a battery (not shown), but this is not a limitation. A detailed description will be given below.

As shown in FIG. 1, the housing 3 is an approximately rectangular parallelepiped and is formed of, for example, a resin such as a PPS resin.

As shown in FIG. 2, the housing 3 is formed by combining a pair of boxes 3a and 3b having an approximately rectangular box shape and each having an opening on one side. The boxes 3a and 3b are combined together in the vertical direction in FIG. 2 such that their openings face each other.

As described later in detail, a metallic surface layer 4 is formed on the inner circumferential surface of the housing 3.

The adjustment unit 5 includes: an approximately rectangular box-shaped metallic case 17 having a flange and an opening toward the upper side (the upper side in FIG. 2); a rectangular frame-shaped seal member (packing) 19 formed of mica and abutting against the flange of the case 17; a conversion section 21 contained in the case 17; and the ceramic wiring board 9.

The flange of the case 17 abuts against the lower surface of the seal member 19, and an outer peripheral portion of the lower surface of the ceramic wiring board 9 abuts against the upper surface of the seal member 19. The opening of the case 17 is thereby closed by the ceramic wiring board 9. The internal space of the closed case 17 forms a first chamber C1.

A pipe-shaped inlet (i.e., an exhalation introduction pipe) 22 and a pipe-shaped outlet 23 that serve as pipe connection ports protrude from the lower surface of the case 17 such that the inlet 22 and the outlet 23 are spaced apart from each other. The inlet 22 and the outlet 23 are in communication with the first chamber C1.

The porous gas-permeable conversion section 21 is disposed within the first chamber C1 to be located between the inlet 22 and the outlet 23. As described later, the conversion section 21 is a structure that functions to convert a first gas component (e.g., NO) contained in exhaled breath to a second gas component (e.g., NO₂).

In this adjustment unit 5, the exhaled breath (G) introduced from the inlet 22 into the first chamber C1 comes into contact with the conversion section 21. As a result, the first gas component in the exhaled breath is converted to the second gas component. Subsequently, the exhaled breath is discharged from the outlet 23 to the gas flow pipe 13.

The sensor unit 7 includes: an approximately rectangular box-shaped metallic case 25 having a flange and an opening toward the lower side; a rectangular flame-shaped seal member 27 formed of mica and bonded to the flange of the case 25; a sensor element section 29 contained in the case 25; a heat insulating sheet 31 formed of a nonwoven fabric of inorganic fibers (e.g., alumina fibers) other than metal fibers; and the ceramic wiring board 9.

The flange of the case 25 is bonded to the upper surface of the seal member 27, and an outer peripheral portion of the upper surface of the ceramic wiring board 9 is bonded to the lower surface of the seal member 27. The opening of the case 25 is thereby closed by the ceramic wiring board 9. The inner space of the closed case 25 forms a second chamber C2.

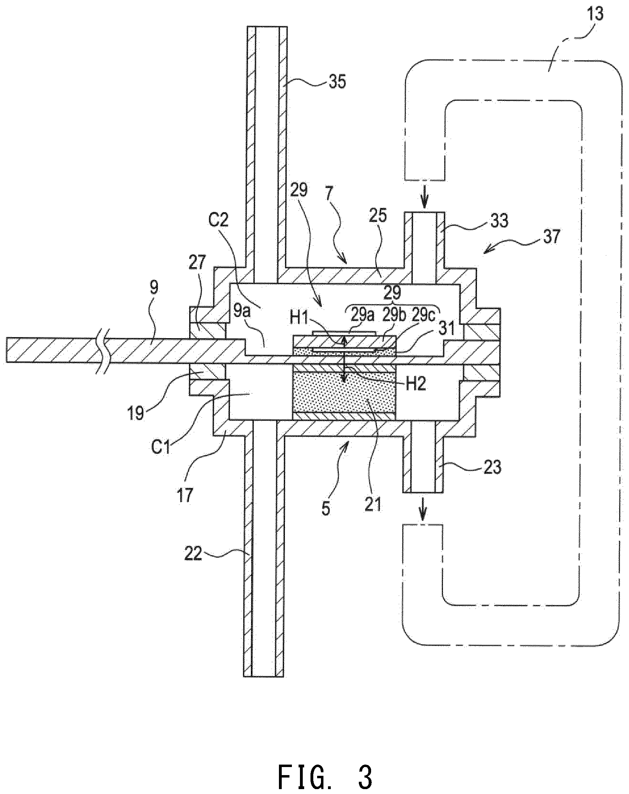

As shown in FIG. 3, the sensor element section 29 has an approximately rectangular plate shape. A sensing section 29a is disposed on the upper surface (on the upper side in FIG. 3) of a base member 29b, and a heater 29c is disposed on the bottom surface of the base member 29b. Specifically, the sensor element section 29 has a stacked structure including the sensing section 29a, the base member 29b, and the heater 29c stacked integrally.

As described later, the sensing section 29a has a mixed potential-type sensor structure, and its electrical characteristic changes with the concentration of the second gas component. The base member 29b is an electrically insulating ceramic substrate formed of, for example, alumina. The heater 29c generates heat upon supply of electricity from a battery (not shown), thereby heating the sensing section 29a to its operating temperature. The heater 29c is a heat-generating resistor which is made of, for example, platinum and is formed on a surface of the ceramic substrate.

A recess 9a is formed in a central portion of the upper surface of the ceramic wiring board 9. The heat insulating sheet 31 is disposed in the recess 9a, and the sensor element section 29 is disposed on the heat insulating sheet 31 such that the heater 29c is in contact with the heat insulating sheet 31.

A pipe-shaped inlet 33 and a pipe-shaped outlet (i.e., an exhalation discharge pipe) 35 protrude from the upper surface of the case 25 such that the inlet 33 and the outlet 35 are spaced apart from each other. The inlet 33 and the outlet 35 are in communication with the second chamber C2.

The sensor element section 29 is disposed in the recess 9a and located between the inlet 33 and the outlet 35 within the second chamber C2.

The gas flow pipe 13 is a pipe made of a resin or a metal. As shown in FIG. 2, a first end of the gas flow pipe 13 is connected to the outlet 23 of the first chamber C1, and a second end of the gas flow pipe 13 is connected to the inlet 33 of the second chamber C2. Specifically, the gas flow pipe 13 allows communication between the first chamber C1 and the second chamber C2 so that the exhaled breath can flow from the first chamber C1 to the second chamber C2.

Although the first and second ends of the gas flow pipe 13 are disposed inside the housing 3, the remaining portion of the gas flow pipe 13 is disposed outside the housing 3 so as to extend along the outer circumferential surface of the housing 3.

Although not shown in the drawings, wiring traces connected to the sensing section 29a and wiring traces connected to the heater 29c are disposed on an end portion (on the left side in FIG. 2) of the ceramic wiring board 9. These wiring traces are connected to unillustrated metallic terminals provided in the first connector portion 11, and the metallic terminals are connected to unillustrated lead wires disposed in the second connector portion 15.

As shown in FIG. 3, the sensor unit 7 and the heater 29c are thermally coupled as indicated by an arrow H1 as a result of the heater 29c being stacked through the sensing section 29a in the sensor unit 7 and the base member 29b.

Similarly, the adjustment unit 5 and the heater 29c are thereby thermally coupled as indicated by an arrow H2 as a result of the heater 29c being stacked on the conversion section 21 in the adjustment unit 5 through part of the ceramic wiring board 9 and the heat insulating sheet 31.

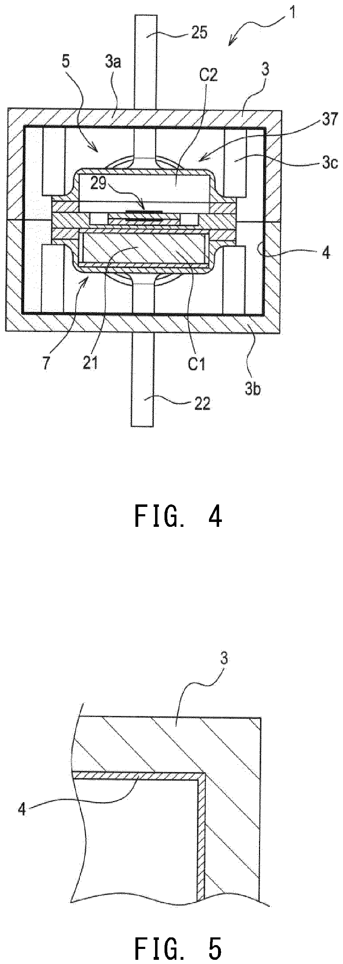

The adjustment unit 5, the sensor unit 7, and the heater 29c are integrated together to form a sensor main body 37. The sensor main body 37 is fixed within the housing 3 by a plurality of engagement members 3c (see FIG. 4) provided within the housing 3 and protruding therefrom.

Specifically, the above-described thermal coupling in the sensor main body 37 allows the single heater 29c to heat the conversion section 21 of the adjustment unit 5 and the sensing section 29a of the sensor unit 7.

The phrase "the sensor unit 7 and the heater 29c are thermally coupled" means that the heater 29c is in direct contact with a component included in the sensor unit 7 with no air therebetween. The meaning of the phase "the adjustment unit 5 and the heater 29c are thermally coupled" is the same as above.

### [1-2. Surface layer]

As shown in FIG. 5, FIG. 2 described above, etc., the surface layer 4 made of a metal such as Ni or Cr is formed over the entire inner circumferential surface of the housing 3.

The surface layer 4 is preferably formed over the entire inner circumferential surface of the housing 3. However, the surface layer 4 may be formed on part of the inner circumferential surface. For example, the surface layer 4 may be formed mainly around the sensor main body 37 that is to be heated to high temperature by the heat generated by the heater 29c.

The performance of the surface layer 4 to reflect radiant heat emitted from the sensor main body 37 is higher than that of the housing 3. Specifically, the reflectivity of the surface layer 4 is within the range of, for example, 0.5 to 0.9 and is higher than the reflectivity of the resin-made housing 3 (e.g., 0.1 to 0.3).

The surface layer 4 can be formed by various well-known methods such as electroless plating, sputtering, and vapor deposition. The surface layer 4 may be formed by applying a metal film (e.g., a metal tape) to the inner circumferential surface of the housing 3.

### [1-3. Flow passage of exhaled breath]

Next, the flow passage of exhaled breath in the exhalation sensor 1 will be described.

As shown by arrows in FIG. 2 etc., the breath (G) exhaled from a person is introduced from the inlet 22 into the first chamber C1, passes through the conversion section 21, and is discharged from the first chamber C1 to the gas flow pipe 13 through the outlet 23.

The exhaled breath is then introduced from the gas flow pipe 13 into the second chamber C2 through the inlet 33. In the second chamber C2, the exhaled breath flows along the sensing section 29a and is discharged to the outside of the second chamber C2 through the outlet 35 (i.e., discharged to the outside of the housing 3).

### [1-4. Operating principle of exhalation sensor]

Next, the operating principle of the exhalation sensor 1 will be described. Since the operating principle is well known as described above, the operating principle will be described briefly.

The conversion section 21 is composed of, for example, a catalyst of Pt-carrying zeolite and is porous so that the exhaled breath can pass therethrough. The catalyst converts the first gas component (e.g., NO) contained in the exhaled breath to the second gas component (e.g., NO₂) at a prescribed ratio (i.e., a prescribed NO/NO₂ partial pressure ratio) at a prescribed activation temperature, i.e., the operating temperature of the catalyst.

The sensing section 29a is configured as a mixed potential NOx (nitrogen oxide) sensor including a solid electrolyte body and a pair of electrodes disposed on the surface of the solid electrolyte body.

For example, the sensing section 29a used may be an element prepared by disposing, on a solid electrolyte body formed of YSZ, a reference electrode formed of Pt and a sensor electrode formed of WO₃.

At the activation temperature of the sensing section 29a, i.e., its operating temperature, different from the activation temperature of the catalyst, the electrical characteristic (electromotive force) of the sensing section 29a changes with the concentration of NOx (i.e., NO₂) contained in the exhaled breath.

Since the heater 29c is disposed close to the sensing section 29a, the sensing section 29a can be heated to the high temperature described above. Meanwhile, the heater 29c is thermally coupled to the conversion section 21 through the heat insulating sheet 31 and the ceramic wiring board 9, and the conversion section 21 can be heated to a temperature different from the temperature of the sensing section 29a.

In this exhalation sensor 1, the concentration of NOx, which is a specific gas component in the exhaled breath, can be detected as follows.

As shown in FIG. 2, the exhaled breath is first introduced from the inlet 22 into the first chamber C1. Since the conversion section 21 has been heated by the heater 29c to the prescribed activation temperature, NO in the exhaled breath is converted to NO₂ at a prescribed partial pressure ratio.

The exhaled breath having undergone the component conversion is discharged from the first chamber C1 to the gas flow pipe 13 through the outlet 23 and then introduced into the second chamber C2 through the inlet 33.

Next, the exhaled breath comes into contact with the sensing section 29a in the second chamber C2, and a potential difference (electromotive force) is generated between the pair of electrodes in accordance with the concentration of NO₂. The concentration of NO₂ can be detected based on the potential difference. The NO₂ is a component converted from NO in the conversion section 21 at the prescribed partial pressure ratio, and the concentration of NO can be determined from the partial pressure ratio.

### [1-5. Effects]

The exhalation sensor 1 of the first embodiment includes the surface layer 4 on its surface facing the sensor main body 37, and the performance of the surface layer 4 to reflect radiant heat is higher than the performance of the housing 3 to reflect radiant heat. Specifically, the radiant heat reflectivity of the surface layer 4 is higher than the radiant heat reflectivity of the housing 3. Therefore, the surface layer 4 can reflect the radiant heat emitted from the sensor main body 37 more efficiently than the housing 3.

In this case, the radiant heat emitted from the sensor main body 37 is unlikely to escape to the outside of the housing 3, so that the heat generated by the heater 29c in the sensor main body 37 can be efficiently stored within the housing 3.

Namely, the heater 29c is used to heat the conversion section 21 and the sensing section 29a to their operating temperatures. When the heat generated by the heater 29c is unlikely to escape to the outside of the housing 3, the temperature inside the housing 3 does not easily decrease. Therefore, the power consumption of the heater 29c for heating the conversion section 21 and the sensing section 29a to their operating temperatures can be reduced.

As described above, in the first embodiment, since the heat can be effectively utilized, a remarkable effect of reducing the power consumption of the heater 29c can be achieved.

In particular, in the case where the exhalation sensor 1 is compact and potable, in general, a compact battery (accordingly, a battery having a small capacity) is used. In the first embodiment, since the power consumption of the heater 29c can be reduced, the consumption of the battery can be reduced. Therefore, a remarkable effect of extending the driven time of the exhalation sensor 1 is achieved.

In the first embodiment, the time from when the exhalation sensor 1 is turned on to when the exhalation sensor 1 starts operating, i.e., the time from when the heater 29c is energized until its operating temperature is reached, can be shortened. This is advantageous because the startup performance of the exhalation sensor 1 is improved.

In the first embodiment, since the housing 3 is made of a resin, advantageously, the housing 3 has a lighter weight and an enhanced heat insulating performance as compared with the case where the housing is made of, for example, a metal. In particular, since the temperature inside the housing 3 does not easily decrease due to the enhanced heat insulating performance of the housing 3, the power consumption of the heater 29c can be further reduced.

Moreover, in the first embodiment, the surface layer 4 is a metal layer and has a higher radiant heat reflectivity (e.g., 0.5 or more) than the resin. Therefore, the radiant heat is less likely to escape to the outside of the housing 3, so that the power consumption of the heater 29c can be further reduced.

### [1-6. Correspondence of terms]

A description will be given of the correspondence between terms in the first embodiment and terms in the present disclosure.

The second chamber C2, the sensing section 29a, the sensor unit 7, the heater 29c, the sensor main body 37, the housing 3, and the surface layer 4 in the exhalation sensor of the first embodiment correspond to examples of the chamber of the sensor unit, the sensing section, the sensor unit, the heater, the sensor main body, the housing, and the surface layer, respectively, in the exhalation sensor of the present disclosure.

### [2. Second embodiment]

Next, a second embodiment will be described. However, the description of the same components as those in the first embodiment will be omitted. The same components as those in the first embodiment are denoted by the same symbols.

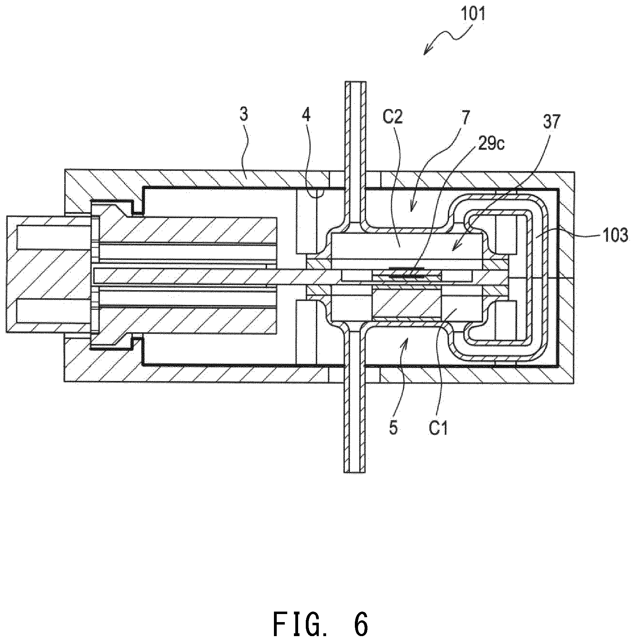

As shown in FIG. 6, in an exhalation sensor 101 of the second embodiment, as in the first embodiment, the sensor main body 37 including the adjustment unit 5, the sensor unit 7, and the heater 29c and other components are disposed in the housing 3.

The metallic surface layer 4 having a higher reflectivity than the material of the housing 3 is formed on the inner circumferential surface of the housing 3.

In particular, in the second embodiment, a gas flow pipe 103 that connects the first chamber C1 to the second chamber C2 is disposed inside the housing 3.

In the second embodiment, the same effects as those in the first embodiment are obtained.

### [3. Third embodiment]

Next, a third embodiment will be described. However, the description of the same components as those in the first embodiment will be omitted. The same components as those in the first embodiment are denoted by the same symbols.

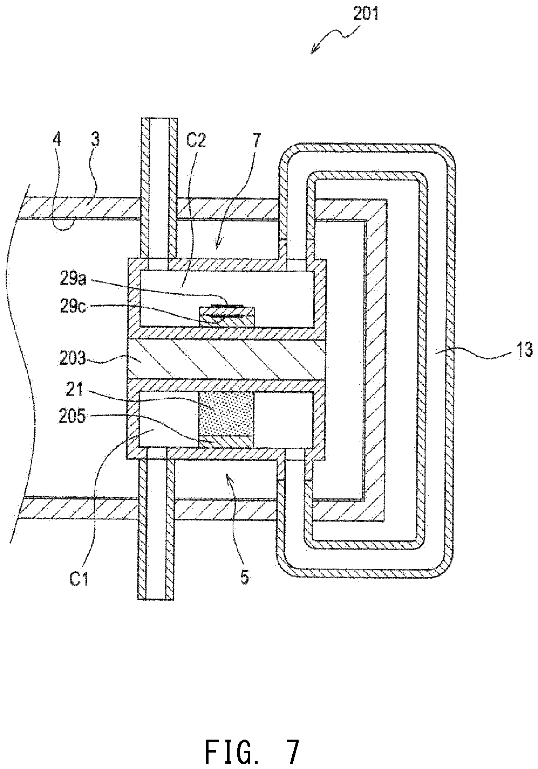

As shown in FIG. 7, in an exhalation sensor 201 of the third embodiment, as in the first embodiment, the housing 3 contains the adjustment unit 5 and the sensor unit 7. The adjustment unit 5 and the sensor unit 7 are disposed with a heat insulator 203 therebetween and are heated by their respective heaters 29c and 205.

Namely, the sensing section 29a is heated by the heater 29c, and the conversion section 21 is heated by the heater 205.

The metallic surface layer 4 having a higher reflectivity than the material of the housing 3 is formed on the inner circumferential surface of the housing 3.

The gas flow pipe 103 that connects the first chamber C1 to the second chamber C2 is disposed mainly outside the housing 3.

In the third embodiment, the same effects as those in the first embodiment are obtained.

### [4. Other embodiments]

The present disclosure is not limited to the embodiments described above, and it will be appreciated that the present disclosure can be implemented in various forms without departing from the present disclosure.
(1) For example, no particular limitation is imposed on the sensor main body. Any sensor main body may be used so long as it includes: a sensor unit including a sensing section whose electrical characteristic (e.g., resistance or electromotive force) changes with the concentration of a specific gas component in a chamber; and a heater that heats the sensing section to its operating temperature, i.e., the temperature at which the change in the electrical characteristic can be detected.
(2) No particular limitation is imposed on the conversion section and the sensing section. The conversion section and the sensing section may have any structures other than those in the first embodiment so long as they have the functions of the present disclosure.
(3) The function of one constituent element in the above embodiments may be distributed to a plurality of constituent elements, or the functions of a plurality of constituent elements may be realized by one constituent element. Part of the configurations of the above embodiments may be omitted. Also, at least part of the configuration of each of the above embodiments may be added to or partially replace the configurations of other embodiments. Notably, all modes included in the technical idea specified by the wording of the claims are embodiments of the present invention.

### DESCRIPTION OF REFERENCE NUMERALS

- 1, 101, 201: exhalation sensor
- 3: housing
- 4: surface layer
- 5: adjustment unit
- 7: sensor unit
- 13, 103: gas flow pipe
- 21: conversion section
- 22, 33: inlet
- 23, 35: outlet
- 29a: sensing section
- 29c, 205: heater
- 37: sensor main body
- C1: first chamber
- C2: second chamber

## Claims

1. An exhalation sensor comprising:
a sensor main body including a sensor unit having a chamber into which exhaled breath is to be introduced and a sensing section whose electrical characteristic changes with the concentration of a specific gas component in the chamber, and a heater for heating the sensing section; and
a housing disposed so as to surround an outer circumference of the sensor main body,
wherein the housing includes a surface layer on a surface thereof that faces the sensor main body, and the surface layer is higher than the housing in terms of the performance of reflecting radiant heat emitted from the sensor main body.

2. An exhalation sensor according to claim 1, further comprising:
an adjustment unit including a chamber into which the exhaled breath is to be introduced and a conversion section that converts a first gas component contained in the exhaled breath introduced into the chamber of the adjustment unit to a second gas component; and
a heater for heating the conversion section,
wherein the chamber of the sensor unit is configured such that the exhaled breath passing through the chamber of the adjustment unit is introduced into the chamber of the sensor unit, and
wherein the electrical characteristic of the sensing section of the sensor unit changes with the concentration of the second gas component in the exhaled breath introduced from the chamber of the adjustment unit.

3. An exhalation sensor according to claim 2, wherein a single heater is used as the heater for heating the conversion section and the heater for heating the sensing section.

4. An exhalation sensor according to any one of claims 1 to 3, wherein the housing is made of a resin.

5. An exhalation sensor according to any one of claims 1 to 4, wherein the surface layer is a metal layer.

6. An exhalation sensor according to claim 5, wherein the surface layer is a plating layer.

7. An exhalation sensor according to claim 5, wherein the surface layer is a film layer formed of a metal film.

8. An exhalation sensor according to any one of claims 1 to 7, wherein the surface layer has a radiant heat reflectivity of 0.5 or more.
